Europäisches Patentamt

European Patent Office

Office européen des brevets

(19)

(11) **EP 1 380 856 A2**

(12) **EUROPEAN PATENT APPLICATION**

(43) Date of publication:
**14.01.2004 Bulletin 2004/03**

(51) Int Cl.[7]: **G02B 1/04**, A61L 27/16

(21) Application number: **03254313.4**

(22) Date of filing: **08.07.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **11.07.2002 JP 2002202969**

(71) Applicant: **Menicon Co., Ltd.
Nagoya-shi Aichi-ken (JP)**

(72) Inventor: **Ichihara, Masuji Menicon Co., Ltd.
Kasugai-shi Aichi-ken (JP)**

(74) Representative: **Paget, Hugh Charles Edward et al
MEWBURN ELLIS
York House
23 Kingsway
London WC2B 6HP (GB)**

(54) **Highly water-absorptive ophthalmic lens and method of producing the same**

(57)     A highly water-absorptive ophthalmic lens which is formed of a macromolecular material including vinyl alcohol unit as a major component, wherein: the macromolecular material is formed by saponifying a copolymer obtained by copolymerization of a polymerizable monomer composition which consists of vinyl acetate and diethylene glycol divinyl ether; the ophthalmic lens has a water content in a range from 73% to 84%; and the ophthalmic lens has a ratio of a dimensional change of less than ±2 % and is free from whitening after (A) the ophthalmic lens has been subjected to three cycles of a freezing-thawing operation wherein the ophthalmic lens formed of the macromolecular material is left at a temperature of not higher than -10°C for not less than twelve hours, and is subsequently left at a temperature in a range from 15°C to 30°C for not less than six hours, and/or (B) the ophthalmic lens has been kept at a temperature in a range from 1°C to 9°C for three months.

EP 1 380 856 A2

**Description**

BACKGROUND OF THE INVENTION

Field of the Invention

The present invention relates to a highly water-absorptive ophthalmic lens such as a contact lens or an intraocular lens, and a method of producing the same. More particularly, the present invention relates to such a highly water-absorptive ophthalmic lens which exhibits a high degree of dimensional stability and is free from whitening or clouding even after the ophthalmic lens has been repeatedly frozen and thawed or even after the ophthalmic lens has been exposed to a low temperature of about 5°C for a long period of time. The present invention also relates to an advantageous method of producing such a highly water-absorptive ophthalmic lens.

Discussion of Related Art

As well known, a contact lens is classified into a soft contact lens and a hard contact lens. Both of the soft and hard contact lenses are needed to have a high degree of permeability to oxygen, so that a sufficient amount of oxygen is supplied to a cornea of an eye on which the contact lens is worn.

The oxygen permeability of the contact lens largely depends on a degree of dissolution of oxygen into a lens material and a degree of mobility or diffusion of oxygen into the lens material. Accordingly, the contact lens which exhibits high degrees of dissolution and diffusion of oxygen into its material exhibits a high degree of oxygen permeability. In a hard contact lens, the oxygen is supplied to the cornea such that the oxygen passes intermolecular spaces or gaps within the lens material. The oxygen permeability of the hard contact lens is improved by increasing the size of the intermolecular spaces or gaps within the lens material, or employing, for the lens material, a component which has a high degree of affinity for the oxygen. In recent years, however, there have been increasing demands for a soft contact lens which assures a lens user of excellent wearing comfort, especially a disposable soft contact lens, in view of a fact that a lens user feels an acute pain in his/her eye if foreign matters get into the eye on which the hard contact lens is worn. In a soft contact lens except the one formed of a silicone material, the oxygen passes through the lens and is supplied to the cornea by a movement of the oxygen dissolved in water contained in the lens. Accordingly, the oxygen permeability of the soft contact lens depends on its water content. Thus, it has been particularly desired to provide a lens having a high degree of water content.

A polyvinyl alcohol-based polymer which includes vinyl alcohol units as a major component in its molecule is considered to be a material suitable for a contact lens material, for the following reasons: The polyvinyl alcohol-based polymer has a high degree of water content, and its alcoholic hydroxyl group (-OH group) as a functional group is neutral and nonionic. Accordingly, the polyvinyl alcohol-based polymer exhibits excellent oxygen permeability, and positively charged calcium ions and protein deposits are less likely to adhere to the polyvinyl alcohol-based polymer. Various kinds of the polyvinyl alcohol-based polymer have been proposed.

One example of the polyvinyl alcohol-based polymer is disclosed in US Patent No. 4,920,180 assigned to the assignee of the present invention and corresponding to JP-A-2-15233, for instance. The disclosed polyvinyl alcohol-based polymer is obtained by saponification of a copolymer of vinyl ester of fatty acid as a major component, (meth) acrylate polymer having at least one polymerizable group per molecule on an average (hereinafter this (meth)acrylate polymer is referred to as "macromonomer"), and a predetermined crosslinking agent. For producing the macromonomer, complicated process steps are required, undesirably pushing up the cost of its manufacture. The macromonomer has at least one polymerizable group per molecule on an average. Accordingly, where the macromonomer has not less than two polymerizable groups per molecule, the macromonomer acts like a crosslinking agent. If such a macromonomer is used in a relatively large amount, the lens to be obtained tends to be fragile or brittle. Since the macromonomer is a monomer having a high molecular weight, there exists a molecular weight distribution, in other words, the macromonomer does not have a constant local molecular weight. Further, the number of the polymerizable groups in each molecule is not constant. Accordingly, the lens formed by using such a macromonomer may not have an intended water content with high stability, but undesirably suffer from a variation in its water content.

Other examples of the polyvinyl alcohol-based polymer are disclosed in JP-A-9-40719 and JP-A-9-40720, respectively. JP-A-9-40719 discloses a contact lens formed of a polyvinyl alcohol-based polymer obtained by saponification of a copolymer of vinyl acetate and triallyl isocyanurate as a crosslinking monomer. JP-A-9-40720 discloses a contact lens formed of a polyvinyl alcohol-based polymer obtained by saponification of a copolymer of vinyl acetate and diallylidene pentaerythritol as a crosslinking monomer. Each of the disclosed contact lenses may suffer from a considerable change in the water content thereof, by slightly changing the amount of the crosslinking monomer to be used, giving rise to a problem in its production process.

Where a contact lens having an intended high water content is formed of the conventional polyvinyl alcohol-based polymers described above, the contact lens may suffer from serious troubles such as a reduction in its size and a whitening or clouding phenomenon after the contact lens has been exposed to a low temperature of about 5° C for a long period of time or after the contact lens has been repeatedly frozen and thawed. It is speculated that the reduction of the size and the whitening phenomenon of the contact lens are caused by crystallization of the polyvinyl alcohol chain. Accordingly, it has been revealed that the contact lens formed of the conventional polyvinyl alcohol-based polymers cannot be used in a winter season.

In an attempt to prevent the crystallization of the polyvinyl alcohol chain, JP-A-6-102471 discloses a water-absorptive contact lens formed of a polyvinyl alcohol-based polymer wherein maleate or N-vinyl lactam is contained as one polymerizable component, for preventing the change of the size of the contact lens by giving an anti-freezing property to the material so as to prevent the crystallization of the polyvinyl alcohol chain. The disclosed contact lens exhibits a high degree of anti-freezing property. In the contact lens wherein the maleate is used as the copolymerizable component, the ester unit of the maleate is hydrolyzed by saponification carried out after copolymerization, so that the carboxylic acid is present in the polymer chain. Therefore, the disclosed contact lens is negatively charged, so that positively charged calcium ions and protein deposits undesirably tend to adhere to the contact lens. The contact lens wherein the N-vinyl lactam is used as the copolymerizable component may be stained with acidic deposits which tend to adhere to the contact lens due to the amide group which is basic. Further, both of the disclosed contact lenses have a considerably low degree of mechanical strength.

SUMMARY OF THE INVENTION

The present invention has been developed in the background art situations described above. It is therefore a first object of the invention to provide a highly water-absorptive ophthalmic lens which exhibits excellent dimensional stability and is free from whitening or clouding after the ophthalmic lens has been repeatedly frozen and thawed or after the ophthalmic lens has been exposed to a low temperature of about 5°C for a long time period, and an advantageous method of producing such a highly water-absorptive ophthalmic lens having the excellent characteristics.

It is a second object of the present invention to provide an ophthalmic lens which exhibits a high degree of anti-staining property by rendering the material of the lens nonionic to prevent generation of anions and cations.

It is a third object of the present invention to provide an ophthalmic lens having a practically satisfactory degree of strength.

It is a fourth object of the present invention to provide a method of producing an ophthalmic lens having the characteristics described above with high stability and at a low cost.

As a result of an extensive study by the inventor of the present invention, it has been found that the problem of crystallization experienced in the ophthalmic lens formed of the conventional polyvinyl alcohol-based polymer is advantageously eliminated or avoided, if an ophthalmic lens is formed of a polyvinyl alcohol-based polymer which is formed of a polymerizable monomer composition consisting of two components, i.e., vinyl acetate as a monomer component that gives vinyl alcohol unit [-CH$_2$-CH(OH)-] and diethylene glycol divinyl ether as a crosslinking monomer (crosslinking agent), and if the water content of the polyvinyl alcohol-based polymer is selected within a range from 73% to 84%. Accordingly, it has been found that the ophthalmic lens formed of the polyvinyl alcohol-based polymer described above exhibits excellent dimensional stability and is free from the whitening phenomenon even after the ophthalmic lens has been repeatedly frozen and thawed or even after the ophthalmic lens has been exposed to a low temperature of about 5°C (in a range from 1° C to 9°C) for a long period of time.

The present invention has been developed based on the findings described above, and the above-indicated objects of the invention may be achieved according to one aspect of the invention which provides a highly water-absorptive ophthalmic lens which is formed of a macromolecular material including vinyl alcohol unit as a major component, wherein: the macromolecular material is formed by saponifying a copolymer obtained by copolymerization of a polymerizable monomer composition which consists of vinyl acetate and diethylene glycol divinyl ether; the ophthalmic lens has a water content in a range from 73% to 84%; and the ophthalmic lens has a ratio of a dimensional change of less than ±2 % and is free from whitening after (A) the ophthalmic lens has been subjected to three cycles of a freezing-thawing operation wherein the ophthalmic lens formed of the macromolecular material is left at a temperature of not higher than -10 ° C for not less than twelve hours, and is subsequently left at a temperature in a range from 15°C to 30°C for not less than six hours, and/or (B) the ophthalmic lens has been kept at a temperature in a range from 1 ° C to 9° C for three months.

The highly water-absorptive ophthalmic lens constructed according to the present invention is formed of the macromolecular material obtained by saponifying the copolymer which is obtained by copolymerization of the polymerizable monomer composition consisting of the vinyl acetate and the diethylene glycol divinyl ether. Further, the highly water-absorptive ophthalmic lens of the present invention has a high degree of water content in a range from 73% to 84%. Therefore, the present highly water-absorptive ophthalmic lens formed of the macromolecular material described above does not suffer from the conventionally experienced problem of crystallization. Accordingly, the present ophthalmic lens is free from serious troubles such as the reduction of the size and the whitening phenomenon even after the ophthalmic lens has been repeatedly frozen and thawed or even after the ophthalmic lens has been exposed to a low temperature of about 5°C for a long period of time. Thus, the present highly water-absorptive ophthalmic lens exhibits excellent dimensional stability and transparency.

It is speculated that the crystallization of the lens is prevented or minimized owing to a reduction in the degree of freedom of the polymer chain by crosslinking, or owing to the polyether structure of the diethylene glycol divinyl ether which is a crosslinking monomer. However, the mechanism is not clear.

Where the diethylene glycol divinyl ether is used, the water content of the macromolecular material does not considerably change even if the amount of the diethylene glycol divinyl ether to be used is slightly changed, contrary to a case where the conventional triallylisocyanurate or diallylidene pentaerythritol is used. However, this mechanism is not clear. Accordingly, the ophthalmic lens to be obtained has the intended water content with high stability.

The macromolecular material for the ophthalmic lens according to the present invention includes at least one alcoholic OH group as the functional group and is nonionic under ordinary wearing conditions of the lens, i.e., in an almost neutral state of the lens, so that negatively charged deposits as well as positively charged calcium ions and protein deposits do not adhere to the ophthalmic lens formed of the macromolecular material.

The present highly water-absorptive ophthalmic lens formed of the macromolecular material does not suffer from a considerable reduction in its strength, unlike an ophthalmic lens formed of the conventional polyvinyl alcohol-based polymer using the above-described maleate, etc. Thus, the present highly water-absorptive ophthalmic lens exhibits a sufficiently high degree of strength required by the ophthalmic lens.

In one preferred form of the present invention, the polymerizable monomer composition consists of 92 to 98.5 wt.% of the vinyl acetate and 1.5 to 8 wt.% of the diethylene glycol divinyl ether. This arrangement permits the ophthalmic lens to have the water content in a range from 73% to 84%.

The above-described objects of the present invention relating to the method of producing an ophthalmic lens may be attained according to another aspect of the invention, which provides a method of producing a highly water-absorptive ophthalmic lens according to the above-described aspect of the invention, comprising the steps of preparing a polymerizable monomer composition which consists of vinyl acetate and diethylene glycol divinyl ether; introducing the polymerizable monomer composition into a mold cavity of a mold assembly which is shaped to produce a configuration of an intended ophthalmic lens; copolymerizing the polymerizable monomer composition by photo-polymerization to obtain a copolymer; and saponifying the obtained copolymer. According to the present method, the intended ophthalmic lens having excellent characteristics such as high degrees of dimensional stability and transparency can be advantageously produced. In addition, since the polymerizable monomer composition is polymerized by the photo-polymerization, the time required for the polymerization can be effectively reduced, as compared in a case where heat-polymerization is employed. Thus, the present method is effective to improve the production efficiency of the ophthalmic lens, for thereby minimizing the cost of its manufacture.

DETAILED DESCRIPTION OF PREFERRED EMBODIMENTS

The highly water-absorptive ophthalmic lens according to the present invention is formed of a macromolecular material, i.e., a polyvinyl alcohol-based polymer formed by saponifying a copolymer which is obtained by copolymerization of a polymerizable monomer composition consisting of two components, i.e., vinyl acetate and diethylene glycol divinyl ether. The water content of the present highly water-absorptive ophthalmic lens formed of the macromolecular material described above is held in a range from 73% to 84%.

If the water content of the ophthalmic lens is less than 73%, the ophthalmic lens does not exhibit an intended high degree of permeability to oxygen, and suffers from a reduction in its strength. If the water content of the ophthalmic lens exceeds 84%, on the other hand, the crystallization of the lens cannot be prevented. In this case, the ophthalmic lens tends to suffer from a reduction in its size and the whitening or clouding phenomenon described above after the ophthalmic lens has been repeatedly frozen and thawed or after the ophthalmic lens has been kept under a low temperature condition for a long time period.

The vinyl acetate as one of the two components which constitutes the polymerizable monomer composition is a major component of the polymerizable monomer composition. The ester bond of the vinyl acetate is hydrolyzed by the saponification treatment carried out after the polymerization to give the vinyl alcohol unit $[-CH_2-CH(OH)-]$. Owing to the hydroxyl group of the vinyl alcohol unit, the water-absorptive property is given to the ophthalmic lens.

Where any one of vinyl propionate, vinyl butyrate, vinyl laurate, and vinyl chloroacetate, each of which is easily hydrolyzed by the saponification carried out after the polymerization to give the vinyl alcohol unit, is used in place of the vinyl acetate, and copolymerized with the diethylene glycol divinyl ether, the saponified ophthalmic lens does not have a sufficiently high degree of elasticity required by the ophthalmic lens. It is speculated that the copolymerizability of each of those monomers with respect to the diethylene glycol divinyl ether used as the crosslinking monomer is deteriorated due to a change in its polymerizability, which change appears to arise from steric hindrance of each monomer, a decrease or an increase of the acid strength in each monomer, etc.

The diethylene glycol divinyl ether which is the other of the two components that constitute the polymerizable monomer composition and which is copolymerized with the vinyl acetate described above is the crosslinking monomer (crosslinking agent) for forming crosslinkages in the macromolecular material for the ophthalmic lens, so that the ophthalmic lens is capable of maintaining its shape with high stability and exhibiting a suitable degree of elasticity.

Where diethylene glycol diallyl ether having a structure similar to that of the diethylene glycol divinyl ether is used in place of the diethylene glycol divinyl ether, and copolymerized with the vinyl acetate, the polymerization does not proceed to a sufficient extent. In this case, the copolymer obtained after the polymerization process remains in a liquid state. Even if the copolymer solidifies, the saponified lens does not have a sufficiently high degree of elasticity required by the ophthalmic lens. It appears that the diethylene glycol diallyl ether has poor copolymerizability with respect to the vinyl acetate.

The amounts of the vinyl acetate and the diethylene glycol divinyl ether to be used are preferably determined such that the amount of the diethylene glycol divinyl ether is 1.5 to 8 parts by weight per 100 parts by weight of the polymerizable monomer composition consisting of the vinyl acetate and the diethylene glycol divinyl ether. In other words, the vinyl acetate is used in an amount of 92 to 98.5 wt. % of the polymerizable monomer composition and the diethylene glycol divinyl ether is used in an amount of 1.5 to 8 wt.% of the polymerizable monomer composition.

If the amount of the diethylene glycol divinyl ether is less than 1.5 wt.%, it is not possible to effectively avoid the problems which appear to arise from the crystallization, such as the reduction in the lens size and the whitening phenomenon of the lens experienced after the lens has been repeatedly frozen and thawed or after the lens has been kept at a low temperature of around 5°C for a long time period. If the amount of the diethylene glycol divinyl ether exceeds 8 wt.%, on the other hand, the water content of the ophthalmic lens to be obtained is less than 70%, so that the ophthalmic lens does not exhibit a satisfactory high degree of oxygen permeability. In addition, the ophthalmic lens tends to be fragile or brittle due to an excessively high crosslinking density.

It is apparent from the EXAMPLES described later that the water content of the lens to be obtained is about 84% where the diethylene glycol divinyl ether is used in an amount of 1.5 wt.% of the polymerizable monomer composition while the water content of the lens is about 73% where the diethylene glycol divinyl ether is used in an amount of 8 wt.%.

In the above-indicated Publications JP-A-9-40719 and JP-A-9-40720, the triallylisocyanurate and the diallylidene pentaerythritol are respectively used as the crosslinking monomer, instead of the diethylene glycol divinyl ether used in the present invention. Even if the triallylisocyanurate or the diallylidene pentaerythritol as the crosslinking monomer is used in an amount of 1.5 to 8 wt.% as specified according to the present invention, the ophthalmic lens to be obtained does not have a water content of not lower than 70%. In addition, the water content of the ophthalmic lens largely changes by slightly changing the amount of the crosslinking monomer to be used.

The vinyl acetate as one of the components which constitute the present polymerizable monomer composition has a boiling point (73°C) which is not so high, so that the vinyl acetate may volatilize. In this case, the proportion of the components of the polymerizable monomer composition may slightly change. It is, therefore, desirable that the water content does not substantially change even if the proportion of the components of the polymerizable monomer composition slightly changes. Where the above-described triallylisocyanurate or diallylidene pentaerythritol is used, however, the water content largely changes with a slight change of the amount of the triallylisocyanurate or diallylidene pentaerythritol to be used. Thus, the ophthalmic lens to be obtained does not have the intended water content with high stability where the triallylisocyanurate or diallylidene pentaerythritol is used as the crosslinking monomer.

In contrast, the diethylene glycol divinyl ether used in the present invention permits the ophthalmic lens to have the intended water content with high stability, irrespective of a slight change in the proportion of the components of the polymerizable monomer composition due to the volatilization of the vinyl acetate.

In producing the ophthalmic lens by using the above-described polymerizable monomer composition which consists of the vinyl acetate and the diethylene glycol divinyl ether, it is possible to add, to the polymerizable monomer composition, various additives conventionally used for the ophthalmic lens, as needed. For instance, a polymerizable UV-absorbing monomer or pigment, or a UV-absorbing pigment may be added to the polymerizable monomer composition for the purpose of imparting the UV-absorbing property to the ophthalmic lens or coloring the ophthalmic lens, so that such UV-absorbing monomer or pigment is introduced into the copolymer as one of the constituent components of the lens. It is needless to mention that each additive should not inhibit the effect of the invention and is added in an amount which does not inhibit the effect of the invention.

For producing the intended ophthalmic lens by using the above-described polymerizable monomer composition, the polymerizable monomer composition is copolymerized into a copolymer, and the obtained copolymer is subjected to a saponification treatment which will be described later.

More specifically described, the polymerizable monomer composition is polymerized by a photo-polymerization method wherein the polymerizable monomer composition to which a sensitizer (which will be described later) has been added is exposed to a suitable light such as an ultraviolet light for polymerization. The photo-polymerization method effectively reduces the time required for the polymerization, as compared with a heat-polymerization, for thereby improving the production efficiency of the ophthalmic lens so as to minimize the cost of its manufacture. As the polymerization system, it is preferable to employ an ordinary bulk polymerization method which assures high polymerization efficiency and high productivity. As needed, a solution polymerization method may be employed.

Examples of the sensitizer include diethoxy acetophenone, 4-(2-hydroxyethoxy)phenyl (2-hydroxy-2-propyl) ketone, 2-hydroxy-2-methyl 1 -phenylpropyl- 1-one, 2-methyl-2-morpholino (4-thiomethylphenyl)propyl- 1-one, 1-hydroxycyclohexyl phenylketone, 2-benzyl-2-dimethylamino 4-morpholinophenyl-butanone, and benzyl dimethylketal.

One or more of those sensitizers may be suitably selected. The amount of the sensitizer to be used is in a range from 0.001 part by weight to 5 parts by weight, preferably in a range from 0.002 part by weight to 3 parts by weight, per 100 parts by weight of the polymerizable monomer composition which consists of the vinyl acetate and the diethylene glycol divinyl ether. If the amount of the sensitizer is less than 0.001 part by weight, the copolymer does not solidify due to a large amount of residual monomers. If the amount of the sensitizer exceeds 5 parts by weight, the copolymer to be obtained does not have a sufficiently large average molecular weight. In this case, the ophthalmic lens does not have a strength required by the ophthalmic lens, or the color of the lens turns yellow.

The copolymer obtained by polymerization described above is then subjected to the saponification treatment, so that the ester unit of the copolymer is hydrolyzed, for thereby providing the hydrophilic ophthalmic lens, in other words, the ophthalmic lens formed of the polyvinyl alcohol-based polymer. The saponification treatment is conducted in a manner similar to the conventional method of saponification of the polyvinyl ester to obtain the polyvinyl alcohol (PVA). Namely, the vinyl acetate unit in the copolymer is treated with an alkaline or an acidic compound, so that the vinyl acetate unit is converted into the vinyl alcohol unit. The saponification with the acidic compound may suffer from the following drawbacks: The saponification speed is comparatively low, and it is difficult to obtain a homogeneous copolymer. Further, the side reaction may take place. In view of this, the saponification with the alkaline compound is preferable in the present invention.

The alkaline compound used for the saponification is a hydroxide of ammonia, an alkali metal or an alkaline earth metal. Examples of the alkaline compound include ammonium hydroxide, sodium hydroxide, potassium hydroxide, and calcium hydroxide. Since these alkaline compounds are usually solid, they are dissolved in a solvent, e.g., alcohols such as methanol, ethanol, propyl alcohol, and butyl alcohol, ethers such as diethyl ether and tetrahydrofuran, or water, so that the alkaline compounds are used for the saponification in the form of alkaline solutions. The copolymer is immersed in the alkaline solution for the saponification. Among those alkaline solutions, those employing alcohols are preferably used. In particular, a 0.1-1.0N alkali alcoholic solution is preferred. For efficient saponification, the alkali alcoholic solution may be mixed with an aqueous alkaline solution.

The reaction temperature for the saponification is generally in a range from 0°C to 70°C. For efficient saponification, the temperature is held in a range from 20°C to 70°C by heating the solution. The saponification at an excessively high temperature may cause deterioration of the ophthalmic lens to be obtained. In view of this, the reaction temperature is preferably not higher than about 70°C. The reaction time for the saponification is suitably determined depending upon the kind and concentration of the alkaline compound, the reaction temperature for the saponification, etc. Practically, it is preferable to determine the kind and concentration of the alkaline compound such that the saponification reaction is completed within a time period from a few minutes to a few hours where the saponification treatment is carried out at room temperature. Further, the saponification reaction may be conducted in the heterogeneous system.

The saponification degree is preferably not less than 90 mol%, more preferably not less than 95 mol%. If the saponification degree is less than 90 mol%, the ophthalmic lens to be obtained may not have the desired water content or the ophthalmic lens may not be used with high stability for a long time period since the water content of the lens and the size of the lens may be changed by a boiling operation, for instance, which is repeatedly conducted on the lens during the use of the lens for a long time period.

The copolymer which has been subjected to the saponification treatment described above is washed with water or saline for neutralization of the residual alkaline compound, or sterilized, so as to provide the water-absorptive ophthalmic lens safe to living bodies.

The ophthalmic lens such as a contact lens or an intraocular lens may be produced, by using the PVA material described above, according to any known methods which include: (1) a mechanical processing method wherein a bar-, block-, or plate-shaped blank (polymer) obtained by polymerization of the polymerizable monomer composition in a suitable mold or vessel is formed into a desired shape by cutting or grinding; (2) a molding method wherein the polymerizable monomer composition is polymerized in a mold cavity of a mold assembly that is shaped to produce the configuration of the intended ophthalmic lens; and (3) a combination of the mechanical processing method and the molding method. Among those methods, the molding method is preferably employed to effectively reduce the cost of manufacture of the lens.

By employing the molding method, a contact lens as the ophthalmic lens is produced in the following manner, for instance. Initially, there is prepared a mold assembly consisting of a male mold and a female mold having respective molding surfaces to give the configuration of the intended contact lens. The polymerizable monomer composition to which the sensitizer necessary for the polymerization has been added is introduced into a mold cavity defined when the male and female molds are closed together. The polymerizable monomer composition accommodated in the mold cavity is exposed to a light such as a UV light for photo-polymerization, whereby the polymerizable monomer composition is polymerized into the copolymer.

The material of the mold assembly is not particularly limited, but it is possible to employ any known materials conventionally used for producing the ophthalmic lenses, as long as at least one of the male and female molds of the mold assembly is formed of a light-transmitting material which permits the light to pass therethrough.

The thus obtained copolymer is removed from the mold assembly according to a known manner, and subjected to the saponification treatment described above, so that the ophthalmic lens according to the present invention is produced. It is needless to mention that the thus produced ophthalmic lens is subjected to a neutralization or a sterilization treatment for assuring the living bodies of a sufficiently high degree of safety.

The ophthalmic lens produced as described above is formed by using the polymerizable monomer composition which consists of the vinyl acetate and the diethylene glycol divinyl ether, and has a water content in a range from 73% to 84%. The present ophthalmic lens exhibits excellent dimensional stability and a high degree of transparency without suffering from whitening or clouding even after the ophthalmic lens has been repeatedly frozen and thawed or even after the ophthalmic lens has been exposed to a low temperature of about 5°C for a long time period, although the reason for this characteristic of the lens is not clear. More specifically described, the ophthalmic lens exhibits excellent dimensional stability and a high degree of transparency after (A) the ophthalmic lens has been subjected to three cycles of a freezing-thawing operation wherein the ophthalmic lens is left at a temperature of not higher than -10 ° C for not less than twelve hours, and is subsequently left at a temperature in a range from 15°C to 30°C for not less than six hours, and/or (B) the ophthalmic lens has been kept at a temperature in a range from 1° C to 9 ° C for three months.

In the ophthalmic lens according to the present invention formed by using the polymerizable monomer composition described above, the diethylene glycol divinyl ether is used as the crosslinking monomer, so that the water content of the ophthalmic lens does not largely change by a slight change of the amount of the diethylene glycol divinyl ether to be used. Therefore, the present ophthalmic lens has the desired water content with high stability.

The macromolecular material for the ophthalmic lens according to the present invention is nonionic, so that the calcium ions and the protein deposits do not tend to adhere to the macromolecular material. Further, the water content of the macromolecular material is held in a range from 73% to 84%, for thereby assuring a sufficiently high degree of strength required by the ophthalmic lens.

EXAMPLES

There will be described some examples of the present invention to further clarify the present invention. It is, however, to be understood that the present invention is not limited to the details of the following examples and the presently preferred embodiments described above, but may be embodied with various changes, modifications and improvements, which may occur to those skilled in the art, without departing from the spirit and scope of the present invention defined in the attached claims.

Preparation and polymerization of polymerizable monomer composition

Initially, there were prepared various polymerizable monomer compositions according to Examples 1-4 of the present invention and Comparative Examples 1 and 2, by mixing vinyl acetate and diethylene glycol divinyl ether in respective amounts as indicated in the following TABLE 1. Further, there were prepared mold assemblies each consisting of a female mold and a male mold formed of polypropylene. To each of the polymerization monomer compositions prepared described above, 2-hydroxy-2-methyl-1-phenyl-propane-1-one was added as the sensitizer. Further, as the coloring matter, tetra(4-methacrylamide)copper phthalocyanine was added, as needed. The thus obtained mixture was filtered by a membrane filter having a pore size of 0.45 $\mu$m. The polymerizable monomer compositions (Examples 1-4 of the present invention and Comparative Examples 1 and 2) to which the sensitizer and the coloring matter as needed had been added were put into the respective female molds. Then, the female molds were assembled with the respective male molds, so that the mold cavities of the respective mold assemblies were filled with the respective polymerizable monomer compositions. The UV light having an intensity of 10 mW/cm$^2$ was applied to the polymerizable monomer compositions by using a high-pressure mercury lamp (2 kW). The polymerizable monomer compositions were exposed to the UV light for 15 minutes, so that the polymerizable monomer compositions were polymerized. Subsequently, the male and the female molds were separated away from each other. The polymerized products in the form of contact lenses were immersed in ethanol, and the contact lenses swollen with ethanol were subsequently removed from the respective molds.

## TABLE 1

| | | | Examples | | | Comparative Examples | |
|---|---|---|---|---|---|---|---|
| | | 1 | 2 | 3 | 4 | 1 | 2 |
| Contents [parts by weight] | VAc | 98.5 | 98.0 | 92.0 | 97.0 | 99.0 | 90.0 |
| | DGDV | 1.5 | 2.0 | 8.0 | 3.0 | 1.0 | 10.0 |
| | Darocure 1173 | 0.4 | 0.4 | 0.7 | 0.4 | 0.4 | 0.7 |
| | APMA | 0.04 | – | – | 0.07 | – | – |

VAc: vinyl acetate

DGDV: diethylene glycol divinyl ether

Darocure 1173: 2-hydroxy-2-methyl-1-phenyl-propane-1-one

APMA: tetra(4-methacrylamide)copper phthalocyanine

Saponification treatment

Each of the swollen contact lenses obtained as described above was immersed in 2 mL of 65% methanol aqueous solution containing 0.5N NaOH accommodated in a vial, and left at room temperature for two hours for saponification. Thereafter, each contact lens was repeatedly subjected to a process wherein the lens was immersed, for 10 minutes, in distilled water whose volume is twenty times as large as the weight of the lens. After it was confirmed that the distilled water in which the lens was immersed turned neutral, each lens was boiled, for 2 hours, in distilled water whose volume is twenty times as large as the weight of the lens. After the lens was cooled, the lens was boiled, for 8 hours, in distilled water whose volume is twenty as large as the weight of the lens, for thereby extracting impurities eluted or released from the lens, such as residual monomers, residual oligomers, non-crosslinked polymers, etc. After each lens was cooled, the lens was put into a heat-resistant bottle filled with 2 ml of saline. Then, the bottle was capped, and subjected to high-pressure steam sterilization at 121°C for 20 minutes.

Measurement of water content

Each of the thus obtained contact lenses (according to Examples 1-4 and Comparative Examples 1 and 2) which had been subjected to the high-pressure steam sterilization described above was immersed in water kept at 20 ° C for two hours. Subsequently, the contact lens was wiped with moisture-absorbing paper to remove redundant aqueous component therefrom. The weight ($W_1$) of the contact lens in its water-absorbing state was measured. Subsequently, the contact lens was left in a drier kept at 60 ° C overnight. The weight ($W_2$) of the contact lens in its dry state was measured. Based on the weight ($W_1$) of the contact lens in the water-absorbing state and the weight ($W_2$) of the contact lens in the dry state, the water content was calculated according to the following equation (I), and the results are indicated in the following TABLE 2.

$$\text{Water content (wt.\%)} = [(W_1 - W_2)/W_1] \times 100 \qquad \text{(I)}$$

Evaluation of resistance to rubbing

For each of the contact lenses according to Examples 1-3 and Comparative Examples 1 and 2, a resistance to rubbing was evaluated in the following manner. To each of the contact lenses placed on a puff, a few drops of cleaning liquid were dripped, and the lens was rubbed with a finger for evaluation of the resistance to rubbing. The criteria of the evaluation of the resistance to rubbing are as follows:

⊚ : The contact lens was not broken even after it was rubbed with the finger, with a relatively large force acting thereon.

○: The contact lens was broken after it was rubbed with the finger, with a relatively large force acting thereon, but was not broken after it was rubbed with the finger, with a relatively small force acting thereon.

(The contact lens is not suitable for a long-term use, but can be used as a disposable lens.)

×: The contact lens was broken after it was rubbed with the finger, with a relatively small force acting thereon.

(The contact lens is not usable even as a disposable lens.)

Evaluation of dimensional stability and transparency

(A) Test 1..... three cycles of freezing-thawing operation

Each of the contact lenses (according to Examples 1-4 of the present invention and Comparative Example 1) which had been subjected to the high-pressure steam sterilization was immersed in water kept at 20°C for two hours. The size ($S_1$) of the contact lens was measured. Subsequently, the contact lens was put into a vial filled with water, so that the contact lens was immersed in water. In this state, the vial was left in a freezer kept at -20°C overnight, whereby the contact lens was frozen. Thereafter, the frozen contact lens in the vial was immersed in water kept at 20°C for eight hours, so that the contact lens was thawed. This freezing-thawing operation was repeated three times.

Each of the contact lenses which had been subjected to the three cycles of freezing-thawing operation was immersed in water kept at 20°C for two hours. In this state, the size ($S_2$) of the contact lens was measured. Based on the size ($S_1$) before the contact lens is subjected to the three cycles of freezing-thawing operation and the size ($S_2$) after the contact lens was subjected to the three cycles of freezing-thawing operation, the ratio of a dimensional change of each contact lens was calculated according to the following equation (II). The ratio of the dimensional change for each contact lens is indicated in the following TABLE 2, together with the size ($S_1$) and the size ($S_2$). It was confirmed by visual observation that the size of the contact lens according to Comparative Example 1 was considerably changed after one cycle of freezing-thawing operation. The size ($S_1$ = 18.2 mm) of Comparative Example 1 was decreased down to 17.5 mm after the one cycle of freezing-thawing operation.

$$\text{Ratio of dimensional change (\%)} = [(S_2 - S_1)/S_1] \times 100 \tag{II}$$

The appearance of each of the contact lenses which had been subjected to the three cycles of freezing-thawing operation was observed with a projector and naked eyes, so as to evaluate the transparency of the contact lens. The criteria of the evaluation of transparency are as follows:

⊚ : The contact lens was colorless and transparent without whitening.

○: Slight whitening was found when observed with the projector, but the contact lens was transparent and no whitening was found when observed with naked eyes.

× : Whitening was found when observed with the projector and naked eyes.

The results of the evaluation are indicated in the TABLE 2.

(B) Test 2····· Storage at low temperature (1°C-9°C) for a long period (three months)

Each of the contact lenses (according to Examples 1-4 of the present invention) which had been subjected to the above-described Test 1 was put into a vial filled with water. Each of the contact lens accommodated in the vial was stored at a temperature of 5°C for three months. After the three-month storage, the contact lens was immersed in water kept at 20°C for two hours. Then, the size ($S_3$) of each contact lens was measured. In each of the contact lenses according to Examples 1-4 of the present invention, the size ($S_3$) after the three-month storage was equal to the size ($S_2$) before the three-month storage. In other words, each of the contact lenses according to Examples 1-4 of the present invention did not suffer from a change in its size even after the three-month storage at the low temperature. The appearance of each of those contact lenses was observed with a projector and naked eyes. No changes were found in the appearance of each contact lens.

## TABLE 2

| | | Water content [%] | Resistance to rubbing | Transparency | | Dimensional stability | | | |
|---|---|---|---|---|---|---|---|---|---|
| | | | | before 3 cycles of freezing-thawing operation | after 3 cycles of freezing-thawing operation | $S_1$ [mm] | $S_2$ [mm] | $S_3$ [mm] | Dimensional change ratio [%] |
| Examples | 1 | 84 | ◎ | ◎ | ○ | 17.1 | 16.8 | 16.8 | −1.8 |
| | 2 | 81 | ◎ | ◎ | ◎ | 16.7 | 16.5 | 16.5 | −1.2 |
| | 3 | 73 | ○ | ◎ | ◎ | 14.5 | 14.6 | 14.6 | 0.7 |
| | 4 | 79 | ◎ | ◎ | ◎ | 16.1 | 16.0 | 16.0 | −0.6 |
| * | 1 | 87 | ◎ | ◎ | × | 18.2 | 17.0 | | −6.6 |
| | 2 | 70 | × | | | | | | |

*: Comparative Examples

$S_1$: the size of the contact lens before the freezing-thawing operation

$S_2$: the size of the contact lens after the three cycles of freezing-thawing operation

$S_3$: the size of the contact lens after the three-month storage at 5°C

It is apparent from the results indicated in the above TABLE 2 that the contact lenses according to Examples 1-4 of the present invention having respective water content values ranging from 73% to 84% did not suffer from substantial changes in the size thereof and exhibited excellent dimensional stability and a high degree of transparency even after the contact lenses were subjected to the three cycles of freezing-thawing operation or even after the contact lenses were exposed to the low temperature for a long time period.

In contrast, the contact lens according to Comparative Example 1 having a water content of 87% suffered from whitening and a reduction in its size as large as 6.0% or higher after the three cycles of freezing-thawing operation. Thus, the contact lens according to Comparative Example 1 is not suitable for practical use under the low-temperature environment. Further, the contact lens according to Comparative Example 2 is not suitable for practical use since the contact lens is too brittle or fragile.

There were prepared contact lenses as Comparative Examples 3-25 which respectively correspond to Examples 1-23 disclosed in US Patent No. 4,920,180 (corresponding to JP-A-2-15233), in a manner similar to that disclosed in the US Patent. Each contact lens has the following specifications: diameter: 14 mm; the center thickness: 0.1 mm; the radius of curvature of its inner surface: 8.5 mm; and the vertex power: -3D.

To evaluate the dimensional stability and transparency of each of the contact lenses according to Comparative Examples 3-25 which respectively correspond to Examples 1-23 of the above-described US Patent 4,920,180 (corresponding to JP-A-2-15233), the above-described "Test 1" [three cycles of freezing-thawing operation] was conducted. The results are indicated in the following TABLE 3.

## TABLE 3

| Comparative Examples | Example No. in USP 4920180 | Transparency | | Dimensional stability | | |
| --- | --- | --- | --- | --- | --- | --- |
| | | before 3 cycles of freezing-thawing operation | after 3 cycles of freezing-thawing operation | $S_1$ [mm] | $S_2$ [mm] | Dimensional change ratio [%] |
| 3 | 1 | ◎ | × | 14.1 | 12.7 | −9.9 |
| 4 | 2 | ◎ | × | 14.0 | 12.5 | −10.7 |
| 5 | 3 | ◎ | × | 14.1 | 13.0 | −7.8 |
| 6 | 4 | ◎ | × | 13.9 | 12.5 | −10.1 |
| 7 | 5 | ◎ | × | 13.9 | 12.9 | −7.2 |
| 8 | 6 | ◎ | × | 14.0 | 12.9 | −7.9 |
| 9 | 7 | ◎ | × | 14.0 | 13.4 | −4.3 |
| 10 | 8 | ◎ | × | 14.0 | 13.1 | −6.4 |
| 11 | 9 | ◎ | × | 13.9 | 13.4 | −3.6 |
| 12 | 10 | ◎ | × | 14.0 | 12.7 | −9.3 |
| 13 | 11 | ◎ | × | 14.0 | 12.7 | −9.3 |
| 14 | 12 | ◎ | × | 13.9 | 12.8 | −7.9 |
| 15 | 13 | ◎ | × | 14.2 | 13.4 | −5.6 |
| 16 | 14 | ◎ | × | 14.0 | 12.7 | −9.3 |
| 17 | 15 | ◎ | × | 13.9 | 12.8 | −7.9 |
| 18 | 16 | ◎ | × | 14.1 | 12.7 | −9.9 |
| 19 | 17 | ◎ | × | 13.9 | 12.6 | −9.4 |
| 20 | 18 | ◎ | × | 14.0 | 12.9 | −7.9 |
| 21 | 19 | ◎ | × | 14.0 | 12.7 | −9.3 |
| 22 | 20 | ◎ | × | 14.1 | 12.8 | −9.2 |
| 23 | 21 | ◎ | × | 13.9 | 13.1 | −5.8 |
| 24 | 22 | ◎ | × | 14.0 | 13.0 | −7.1 |
| 25 | 23 | ◎ | × | 14.0 | 13.0 | −7.1 |

$S_1$: the size of the contact lens before the freezing-thawing operation

$S_2$: the size of the contact lens after the three cycles of freezing-thawing operation

It is apparent from the results indicated in the above TABLE 3 that the contact lenses formed of the conventional polyvinyl alcohol-based polymer suffered from whitening and a reduction in the size thereof as large as 3.6% or higher. Thus, the conventional contact lenses are not suitable for practical use under the low-temperature environment.

As is apparent from the description described above, the highly water-absorptive ophthalmic lens according to the present invention is formed of the macromolecular material, i.e., polyvinyl alcohol-based polymer which is obtained by saponifying a copolymer obtained by copolymerization of the polymerizable monomer composition consisting of the vinyl acetate and the diethylene glycol divinyl ether. Further, the water content of the highly water-absorptive ophthalmic lens of the present invention is selected within a range from 73% to 84%. Accordingly, the crystallization of the ophthalmic lens can be effectively minimized or avoided. Therefore, the present ophthalmic lens exhibits excellent dimensional stability and a high degree of transparency without suffering from a reduction in its size and the whitening or clouding even after the ophthalmic lens has been repeatedly frozen and thawed or even after the ophthalmic lens has been exposed to a low temperature of about 5 ° C for a long period of time.

The macromolecular material for the present ophthalmic lens is nonionic, so that the negatively charged deposits as well as the positively charged calcium ions and protein deposits do not tend to adhere to the macromolecular material, i.e., the ophthalmic lens. Accordingly, the ophthalmic lens exhibits an excellent anti-staining property.

In the polymerizable monomer composition used for producing the present ophthalmic lens described above, the diethylene glycol divinyl ether used as the crosslinking agent is included in the prescribed amount to permit the ophthalmic lens to have the intended water content in a range from 73% to 84%. Accordingly, the ophthalmic lens has a sufficiently high degree of strength required by the ophthalmic lens.

According to the present method of producing a highly water-absorptive ophthalmic lens described above, the highly water-absorptive ophthalmic lens which exhibits excellent dimensional stability and a high degree of transparency can be produced with high stability and high efficiency, for thereby minimizing the cost of manufacture of the ophthalmic lens.

The present invention also extends to the lens of the invention as defined and described herein, in its dried state (containing substantially no water) or in a state in which it contains water in an amount less than its maximum capacity for water.

## Claims

1. A highly water-absorptive ophthalmic lens which is formed of a macromolecular material including vinyl alcohol unit as a major component, **characterized in that**:

   the macromolecular material is formed by saponifying a copolymer obtained by copolymerization of a polymerizable monomer composition which consists of vinyl acetate and diethylene glycol divinyl ether;
   the ophthalmic lens has a water content in a range from 73% to 84%; and
   the ophthalmic lens has a ratio of a dimensional change of less than ±2 % and is free from whitening after (A) the ophthalmic lens has been subjected to three cycles of a freezing-thawing operation wherein the ophthalmic lens formed of the macromolecular material is left at a temperature of not higher than -10°C for not less than twelve hours, and is subsequently left at a temperature in a range from 15°C to 30°C for not less than six hours, and/or (B) the ophthalmic lens has been kept at a temperature in a range from 1°C to 9°C for three months.

2. A highly water-absorptive ophthalmic lens according to claim 1, wherein the polymerizable monomer composition consists of 92 to 98.5 wt.% of the vinyl acetate and 1.5 to 8 wt.% of the diethylene glycol divinyl ether.

3. A highly water-absorptive ophthalmic lens according to claim 1 or 2, wherein the macromolecular material is nonionic.

4. A highly water-absorptive ophthalmic lens according to any one of claims 1 to 3, wherein the diethylene glycol divinyl ether is a crosslinking monomer for forming crosslinkages in the macromolecular material.

5. A highly water-absorptive ophthalmic lens according to any one of claims 1 to 4, wherein the copolymer is saponified at a temperature in a range from 0°C to 70 ° C.

6. A method of producing a highly water-absorptive ophthalmic lens according to any one of claims 1 to 5, comprising the steps of:

   preparing a polymerizable monomer composition which consists of vinyl acetate and diethylene glycol divinyl ether;
   introducing the polymerizable monomer composition into a mold cavity of a mold assembly which is shaped to produce a configuration of an intended ophthalmic lens;
   copolymerizing the polymerizable monomer composition by photo-polymerization to obtain a copolymer; and
   saponifying the obtained copolymer.

7. A method according to claim 6, wherein the mold assembly consists of a male mold and a female mold at least one of which is formed of a light-transmitting material.

8. A method according to claim 6 or 7, wherein at least one sensitizer is added to the polymerizable monomer composition.

**9.** A method according to claim 8, wherein the at least one sensitizer is added to the polymerizable monomer composition in an amount of 0.001 to 5 parts by weight per 100 parts by weight of the polymerizable monomer composition.